# EUROPEAN PATENT APPLICATION

(11) **EP 0 672 427 A1**
(43) Date of publication of application: **20.09.1995**
(21) Application number: 94104243.4
(22) Date of filing: 17.03.1994
(51) Int. Cl.: A61M 5/142

(54) **System for infusion of medicine into the body of a patient**

(71) Applicant: Siemens Elema AB, S-171 95 Solna 1 (SE); SIEMENS AKTIENGESELLSCHAFT, D-80333 München (DE)
(72) Inventor: Pfeiler, Manfred, Dr., D-91054 Erlangen (DE); Mund, Konrad, Dr., D-91080 Uttenreuth (DE); Preidel, Walter, Dr., D-91058 Erlangen (DE); Sjöholm, Gösta, S-17800 Ekerö (SE)

(57) **Abstract**

A system for infusion of medicine into the body of a patient comprises an implantable infusion apparatus including a dosing unit (10) with a reservoir for the medicine and a medicine delivery pump for pumping doses of the medicine from the reservoir into the patient. The infusion apparatus also includes a sensor (2, 4, 6) for sensing a parameter of the patient for controlling the dosing of medicine according to the sensed parameter, and telemetering communication means (8, 12) for communication with an external controller (14). The dosing unit and the sensor are operatively separable.

## Description

The present invention relates to a system for infusion of medicine into the body of a patient comprising an implantable infusion apparatus including a dosing unit with a reservoir for the medicine and a medicine delivery pump for pumping doses of medicine from the reservoir into the patient, a sensor for sensing a parameter of the patient for controlling the dosing of medicine according to the sensed parameter, and telemetering communication means for communication with an external controller.

Systems of this kind for infusion of e.g. insulin are previously known, see e.g. W. Schubert et al, "An implantable artificial pancreas", Medical and Biological Engineering & Computing, 1980, 18, pp. 527-537. In this document an artificial implantable pancreas is described in which, in a first mode of operation, a glucose sensor is transmitting the actual blood glucose level to a control unit, in which the amount of insulin to be infused is calculated on the basis of patient specific parameters, recorded in a program memory and corresponding control signals for a dosing unit is determined by a control algorithm. If no sensor is used or if the sensor employed fails the dosing unit is controlled, in a second mode of operation, by a stored dosing program. Thus, the first mode of operation corresponds to a closed control loop and the second mode of operation to an open control loop.

In US-A-4,543,955 a system is also described comprising an implantable physiological sensor for controlling an implantable device in response to changes in the parameter sensed by the sensor. The implantable device can be a heart pacing device, a drug infusion pump or another device acting upon a human body. In this system the sensor is directly communicating with the device by conductors or a telemetry link. A heart pacing system of this kind is also described in EP-A1-0,317,986.

Considerable efforts are made in developing implantable closed loop systems for the control of blood glucose in which a sensor is continuously monitoring the glucose level and controlling in an optimal way the infusion of insulin into the patient. The construction of such an implanted system, which is completely safe to the patient, has appeared not to be without difficulties.

The purpose of the present invention is to propose a new system for infusion of medicine into the body of a patient in which the difficulties related to the construction of the above mentioned systems are avoided.

This purpose is obtained with a system of the kind defined in the introduction and having the characterizing feature of claim 1.

With the system according to the invention several important advantages are obtained. Thus, the operation of the sensor and the dosing unit can be easily checked individually. The sensor can easily be disconnected if it should fail and the dosing unit can be operated according to a dosing program or by manual control. The initial adjustment and calibration of the system and the development of an algorithm for controlling the dosing in response to the sensed parameter can be easily performed by analyzing sensor data and selecting the dosing of medicine correspondingly. The system according to the invention is particularly well suited for determining an algorithm from the sensor data at the beginning of the operation of the system as the physician and/or the patient can easily change the controlling and adapt the insulin dosing to the sensed data according to experience whereupon this first "approximation" is successively improved and adapted to the real needs of the patient based on the reaction of the body of the patient. It is also easy for the physician or the patient to intervene and change the control later on. Further, as the function of the sensor and the dosing unit can be easily checked, reliable function of the system as a whole can be easily guaranteed too.

According to an advantageous embodiment of the system according to the invention the external controller comprises telemetering communication means for selectable communication with the dosing unit or the sensor or both simultaneously. In this way data can be received from the sensor and the dosing unit separately and an injection schedule can be determined based on telemetered sensor data and telemetered dosing unit data.

According to another advantageous embodiment of the system according to the invention the telemetering communication means of the external controller, the dosing unit and the sensor are constructed for two-way communication between the external controller and each one of the dosing unit and the sensor. In this way program data can be transferred from the external controller to the sensor and sensed data and calibration data can be delivered by the sensor to the external controller. Further, the dosing unit can receive injection schedule data, safety related data, and system indicating data and it can deliver patient related data, operation data of the dosing unit and injection reports to the external controller.

According to yet another advantageous embodiment of the system according to the invention the dosing unit and the sensor are mechanically separated. The dosing unit and the sensor can then be implanted at different times and at different sites in the body of the patient. In certain applications it is desirable to measure the relevant parameter in one point of the body and inject the medicine in another.

According to another further development of the system according to the invention the external controller is portable by the patient. Then a continous connection between dosing unit and the sensor can be maintained by way of the external controller and according to a further development of the system according to the invention the dosing can be automatically controlled by the sensor. Thus, in this way a closed control loop regulation is created.

According to another advantageous embodiment of the system according to the invention the sensor is switchable by the external controller between a passive state and an active state, the sensor performing its functions in the active state. In this way the power consumption of the sensor is reduced.

According to further developments of the system according to the invention the external controller can calculate an injection schedule for the dosing unit from data received from the sensor and possibly also the dosing unit. This calculation can be done in different ways, like according to a stored algorithm or computer program, a fuzzy logic system or a neuronal network system. The external controller can also comprise means for proposing to the patient a new or amended injection schedule for acceptance or modification by the patient before being transmitted as injection schedule to the dosing unit.

According to yet another advantageous embodiment of the system according to the invention the external controller comprises means for indicating to the patient information received from the sensor and the dosing unit. Such indicating means could be a display, beeper means etc.

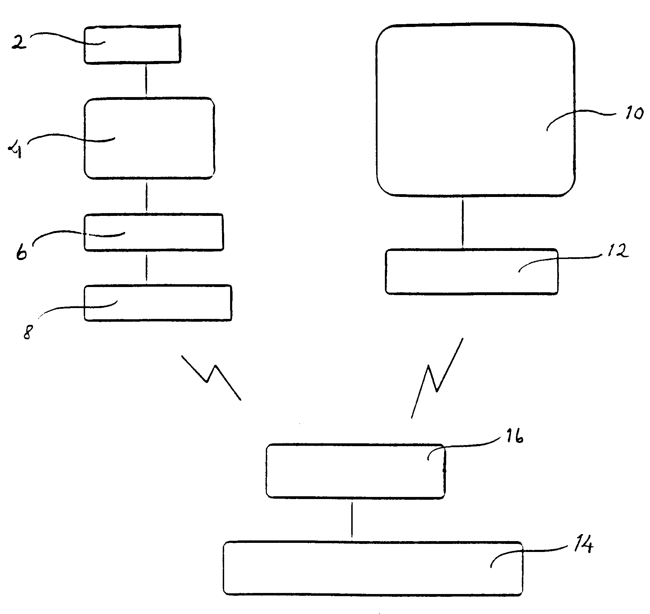

An embodiment, chosen as an example, will now be described more in detail with reference to the enclosed drawing.

On the drawing an embodiment of the system according to the invention is shown, comprising an implantable sensor 2 with associated data processing means and memories 4, telemetry processing means 6 and telemetering communication means 8. The system further comprises an implantable dosing unit 10 with a reservoir for the medicine to be infused into the body of a patient and a medicine delivery pump for pumping doses of the medicine from the reservoir into the patient. The dosing unit 10 further comprises electronics for the control of its function and a power source, in the form of a battery, for its operation. These individual parts of the dosing unit 10 are not shown separately in the drawing as they are well-known to the man skilled in the art and do not *per se* form any part of the invention.

The dosing unit 10 is also provided with telemetering communication means 12.

The system further comprises an external controller 14 with associated telemetering communication means 16 for communication with the sensor 2, 4, 6 and the dosing unit 10 via their telemetering communication means 8 and 12 respectively.

The telemetering communication means 8, 12, 16, of the sensor, the dosing unit, and the external controller are each provided with sending and receiving means for two-way communication between the external controller 14 and each one of the dosing unit 10 and the sensor 2.

The sensor 2 can be an insulin sensor sensing the blood sugar level of the patient. These sensed data can be transmitted continuously to the external controller 14 which then controls the dosing unit 10 to deliver insulin accordingly. The data sensed by the sensor 2 can also be stored in a memory in the part 4 and read out on command from the external controller 14. The sensor 2 can also deliver calibration data to the external controller 14.

The sensor can also receive program data from the external controller 14 for storage in the memories of the part 4.

The sensor is switchable by the external controller 14 between an active state in which the parameter of interest is sensed, like the blood sugar level, and in which information is received and sent to the external controller 14 and a passive state in which the energy consumption is a minimum. In this way the total power consumption of the implant is reduced.

Also the dosing unit 10 is switchable by the external controller 14 between an active and a low energy consuming passive state. In its active state the dosing of insulin is performed and information is sent to and received from the external controller via the telemetering communication means 12, 16. Thus, the dosing unit 10 can receive e.g. injection schedule data from the external controller 14. It can receive and send safety related data, patient related data, operation data, and system identification data. Further, reports of successful injections of insulin can be delivered from the dosing unit 10 to the external controller 14 for comparison with predetermined limiting values such that the controlling of the dosing unit 10 can be changed when such a limiting value is reached. The dosing unit 10 is also disposed to confirm receipt of instructions from the external controller 14 for safety reasons.

The external controller 14 contains (not shown) data entry means, data information means, like a display or beeper means for providing information to the patient, communication means and data storage means. It can selectively activate the sensor and the dosing unit for receiving and sending information as described above and calculate injection schedules based on telemetered sensor data according to a stored algorithm, a computer program or by means of a fuzzy logic system or a neuronal network system.

The injection schedule can be based on telemetered sensor data and telemetered pump data from the dosing unit 10, or be calculated based on data received from the sensor and a programmed patient dosing plan.

The external controller also has means such as switches, buttons, microphones etc (not shown) for receiving information from the patient, and can be provided with means for sending and receiving data from other external apparatus like an external computer.

As mentioned above the dosing unit 10 can be automatically controlled by the external controller 14 in accordance with data received from the sensor 2 in a closed-loop regulation. The external controller 14 is then permanently carried by the patient. The sensor 2 can, however, be disconnected from the external controller 14 and the dosing unit 10 be controlled manually or according to a predetermined dosing program. This is of value if the sensor should fail or other problems with the sensor circuit should appear.

The system according to the invention can also be provided with means for direct communication between the sensor 2 and the dosing unit 10, either telemetering communication means or a galvanic connection. The system is then switchable between one mode of operation with direct communication between the sensor and the dosing unit and another mode of operation in which this direct connection is interrupted and communication between sensor and dosing unit takes place through the external controller.

Above embodiments of the system according to the invention have been described for insulin therapy. However, the system according to the invention can be used for infusion of also other kinds of medicines. Thus, the system according to the invention can be used for e.g. blood pressure patients or patients having circulatory disorders for providing them with medicines. The sensor is then formed of a sensor for sensing blood pressure or a peripherally implanted oxygen sensor.

## Claims

1. A system for infusion of medicine into the body of a patient comprising an implantable infusion apparatus including a dosing unit (10) with a reservoir for the medicine and a medicine delivery pump for pumping doses of the medicine from the reservoir into the patient, a sensor (2, 4, 6) for sensing a parameter of the patient for controlling the dosing of medicine according to the sensed parameter, and telemetering communication means (8, 12) for communication with an external controller (14, 16), **characterized** in that the dosing unit (10) and the sensor (2, 4, 6) are operatively separable.

2. The system according to claim 1, **characterized** in that the dosing unit (10) and the sensor (2, 4, 6) are galvanically separable and are each provided with separate telemetering communication means (12, 8).

3. The system according to claim 2, **characterized** in that the telemetering communication means (12, 8) of the dosing unit (10) and the sensor (2, 4, 6) are devised for communication with the external controller (14).

4. The system according to claims 2 or 3, **characterized** in that the external controller comprises telemetering communication means (16) for selectable communication with the dosing unit (10) or the sensor (2, 4, 6) or both simultaneously.

5. The system according to any one of the claims 2 through 4, **characterized** in that the telemetering communication means (16; 12; 8) of the external programmer (14), the dosing unit (10) and the sensor (2, 4, 6) are constructed for two-way communication between the external controller and each one of the dosing unit and the sensor.

6. The system according to any one of the claims 1 through 5, **characterized** in that the dosing unit (10) and the sensor (2, 4, 6) are mechanically separated.

7. The system according to any one of the claims 1 - 6, **characterized** in that the external controller (14) is portable by the patient.

8. The system according to claim 7, **characterized** in that the dosing unit (10) and the sensor (2, 4, 6) are connectable via their respective telemetering communication means and the external controller (14) for automatic control of the dosing by the sensor.

9. The system according to any one of the claims 1 through 8, **characterized** in that the sensor (2, 4, 6) is switchable by the external controller (14) between a passive state and an active state in which said parameter is sensed.

10. The system according to any one of the claims 1 through 9, **characterized** in that the sensor (2, 4, 6) comprises a memory for storing sensed parameter data, said stored data being transmitted to the external controller (14) upon request from the external controller.

11. The system according to any one of the claims 1 through 10, **characterized** in that the external controller (14) is devised to calculate an injection schedule for the dosing unit (10) from parameter data received from the sensor (2, 4, 6).

12. The system according to any one of the claims 1 through 11, **characterized** in that the external controller (14) comprises means for proposing to the patient a new or amended injection schedule, based on parameter data received from the sensor (2, 4, 6), for acceptance or modification by the patient before being transmitted as injection schedule to the dosing unit (10).

13. The system according to any one of the claims 1 through 11, **characterized** in that the external controller (14) is devised to calculate the injection schedule based on data received from the sensor (2, 4, 6) and a programmed patient dosing plan.

14. The system according to any one of the claims 1 through 11, **characterized** in that the external controller (14) is devised to calculate an injection schedule for the dosing unit (10) from data from the sensor (2, 4, 6) and the dosing unit (10).

15. The system according to any one of the claims 1 through 14, **characterized** in that the external controller (14) comprises means for indicating to the patient information received from the sensor (2, 4, 6) and the dosing unit (10).

16. The system according to any one of the claims 1 through 15, **characterized** in that thet external controller (14) is devised to calculate the injection schedule according to a stored algorithm or computer program.

17. The system according to any one of the claims 1 through 15, **characterized** in that the external controller (14) comprises a fuzzy logic system or a neuronal network system for the calculation of the injection schedule.
